# EUROPEAN PATENT SPECIFICATION

(11) **EP 1 320 399 B1**
(45) Date of publication and mention of the grant of the patent: **30.11.2005**
(21) Application number: 01970003.8
(22) Date of filing: 26.09.2001
(51) Int. Cl.: A61M 15/00

(54) **DEVICE FOR DISPENSING PARTICULATE MATERIAL**
VORRICHTUNG ZUR VERABREICHUNG VON STOFFEN IN PARTIKELFORM
DISPOSITIF D'ADMINISTRATION D'UNE SUBSTANCE PARTICULAIRE

(30) Priority: 27.09.2000 GB 0023653
(43) Date of publication of application: 25.06.2003
(73) Proprietor: Merck Patent GmbH, 64293 Darmstadt (DE)
(72) Inventor: Reber, Dominic Charles, CH-1800 Vevey (CH); Newton, Michael Edgar, Wattisfield, Nr Diss Norfolk IP22 1NS (GB); Hammond, Christopher John, Lode Cambridgeshire CB5 9EN (GB); Charbonnier, Pierre, F-06000 Nice (FR); Michaud, Gary Lionel, Napa, CA 94558 (US); McArthur, Peter R., Danville, CA (US)
(74) Representative: Roberts, David Leslie
(86) International application number: PCT/GB2001/004314
(87) International publication number: WO 2002/026303

(56) References cited:
- WO-A-91/06333
- WO-A-95/31238
- US-A- 5 301 666
- US-A- 5 415 162

## Description

This invention relates to devices for dispensing a plurality of doses of particulate material, and in particular to inhalers for use in the self administering of a pharmacologically active substance in powder form by inhalation.

### Background to the Invention

Inhalers for dispensing a medicament in a dry powder form are becoming increasingly common, and in many cases are intended for use with containers having a number of compartments, each holding a respective dose of medicament, and sealing means, for example a laminated foil seal for hermetically sealing each dose in its compartment. Such inhalers include a mechanism for piercing the seal to enable the medicament to be released from a compartment, and an indexing mechanism which moves the container relative to the inhaler to bring each compartment in turn into registry with an airway, through which the user inhales.

In order to operate such devices properly, the indexing mechanism must be operated correctly, otherwise (for example) it is possible that the compartment in registry with the airway has not had its seal broken when the user inhales, or has previously been emptied or that a compartment is indexed out of registry with the airway before its dose has been dispensed.

### Summary of the Invention

According to the invention, there is provided a device for dispensing a plurality of doses of particulate material from a container having a plurality of compartments, each for holding a respective dose or part thereof, the device comprising an airway extending from the portion of the device for receiving the container to an outlet, indexing means for moving a container received by the device relative to the airway so as to bring successive compartments into registry with the latter, a control member movable through a series of alternating advance and return strokes to operate the indexing means, wherein the device includes a non-return mechanism for preventing movement of the control member in the direction of each return stroke before the completion of the respective preceding advance stroke.

Thus, the invention ensures that the control member is moved to a sufficient extent properly to operate the indexing means. Thus, if a user inadvertently, fails to move the control member through a full advance stroke, the non-return mechanism prevents the user from returning the control member to its original position, and thus from obtaining the incorrect impression that the indexing means has been properly operated.

Preferably, the control member is so linked to the indexing means that, in use, said movement of the container is caused by the return strokes of the control member.

The device may to advantage be adapted for use with a container in which each dose is sealed in its compartment, in which case the device preferably includes opening means for breaking or opening the seal on each compartment, wherein the opening means is also linked to the control member so that operation of the latter operates both the indexing means and opening means.

In this case, the non-return mechanism not only ensures that the indexing means is properly operated, but also that the indexing means and opening means are operated in the correct sequence.

The opening means may conveniently comprise a piercing member so linked to the control member that each advance stroke of the latter extends the piercing member from its retracted position, in which it is situated clear of a compartment into an extended position in which it has pierced that compartment's seal.

Preferably, the device includes a holding member so linked to the control member as to be extended by each advance stroke of the latter to engage a container held in the device and thereby hold a compartment in registry with the airway.

The holding member thus helps to ensure that the compartment does not move out of registry with the airway prematurely, as a result of (for example) sudden movement of the inhaler.

Preferably, the non-return mechanism is also operable to prevent movement of the control member in the direction of each advance stroke until the end of the respective preceding return stroke has been reached. This ensures that the control member is in its proper starting position before each cycle of operation of the device.

Preferably, the advance and return strokes of the control member are constituted by rotational movements of the latter relative to a body of the device, the non-return mechanism comprising a pawl means and a set of teeth, each mounted on a respective one of the control member and the body. Conveniently, the teeth are mounted on the body and the carrier on the control member.

Preferably, the pawl means comprises a pair of opposed pawls mounted on a movable carrier, one pawl engaging the teeth on each advance stroke, the other on each return stroke, the mechanism including abutment means for moving one pawl into engagement with the teeth and disengaging the other pawl at the end of each stroke.

The invention also lies in an inhaler comprising a device as herein above described.

Preferably, the control member of the inhaler includes a cover for covering the outlet of the airway when the inhaler is not in use, wherein the cover is moved clear of the outlet by each advance stroke of the control member.

The invention also lies in a housing for a device/inhaler as herein above described, the housing having receiving means for receiving a container of particulate material, means for receiving indexing means, an outlet for the airway of the container, the housing also carrying a control member for operating the indexing means and means for linking the control member to an indexing means contained, in use, within the housing, wherein the control member is movable through a series of alternating advance and return strokes, and there is provided a non-return mechanism for preventing the movement of the member in the direction of each return stroke before the completion of the respective preceding advance stroke.

### Brief Description of the Drawings

The invention will now be described, by way of example only, with reference to the accompanying drawings, in which:
Figure 1 is an exploded isometric view of an inhaler in accordance with the invention;
Figure 2 is a similarly exploded view of the inhaler when rotated through 180° relative to Figure 1;
Figure 3 is a further exploded view of the inhaler, showing certain components in an assembled form;

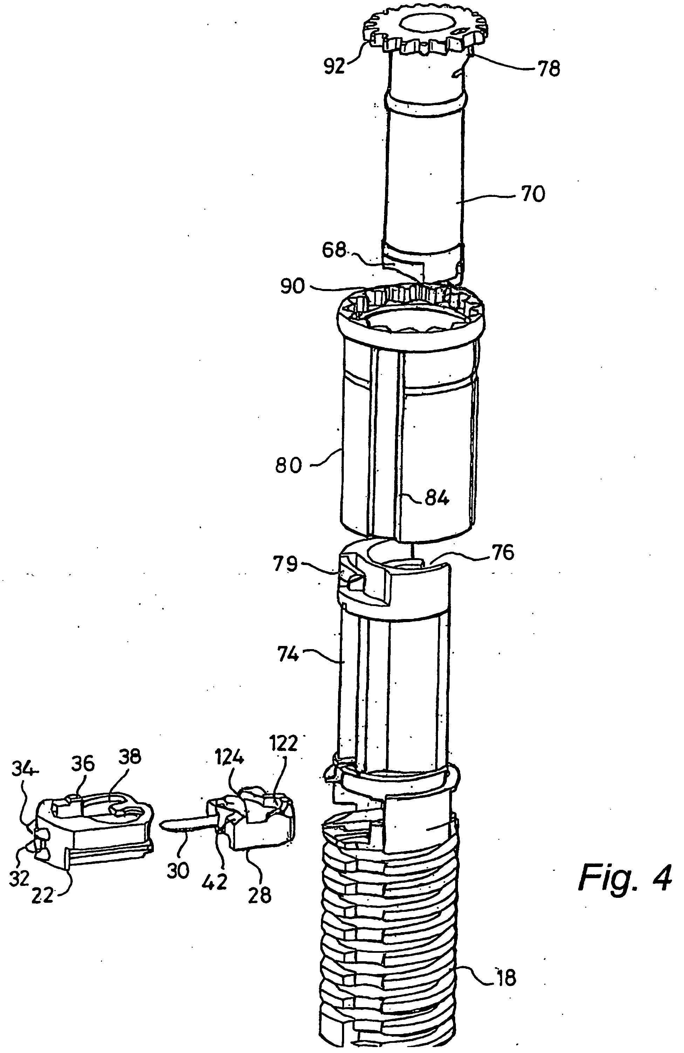

Figure 4 is an exploded view of those components;
Figure 5 is a sectional side view of the inhaler;

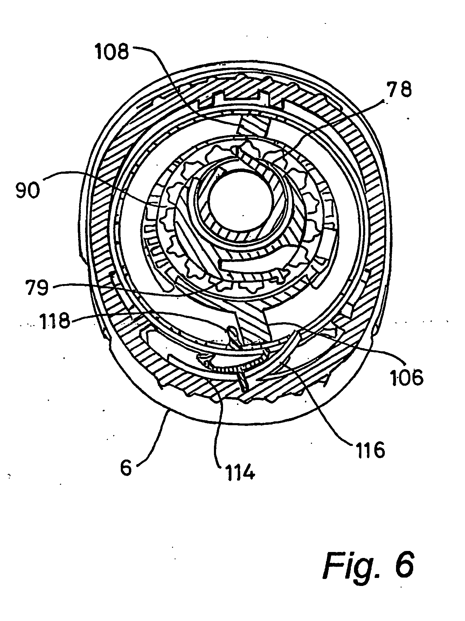

Figure 6 is a sectional view taken along the line H-H of Figure 5;

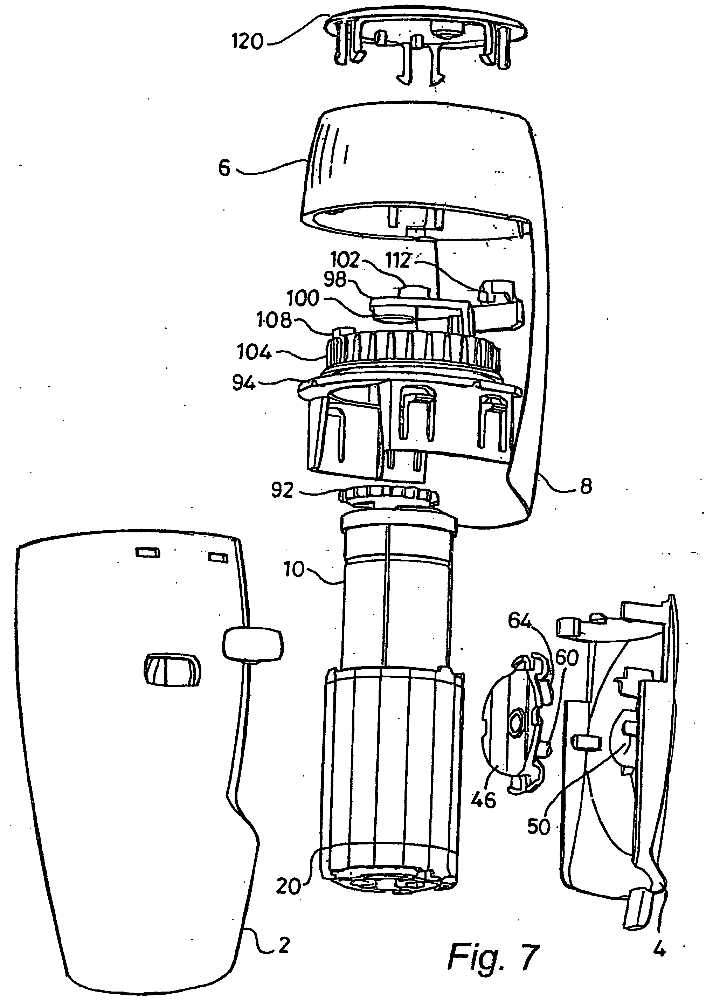

Figure 7 is a similar view (from a slightly different angle) to Figure 3;
Figures 8A-D to Figure 14A-D are cut-away end views of the inhaler during various stages in one cycle of its operation;
Figure 15 is a perspective view, from a different angle, of one of the components shown in Figure 4;
Figure 16 is a further perspective view of another component of the inhaler;
Figure 17 is a perspective view of the inhaler, when assembled and with its mouthpiece uncovered; and
Figure 18 is a graph illustrating the relationship between pressure drop in the inhaler and the rate of airflow therethrough.

### Detailed Description

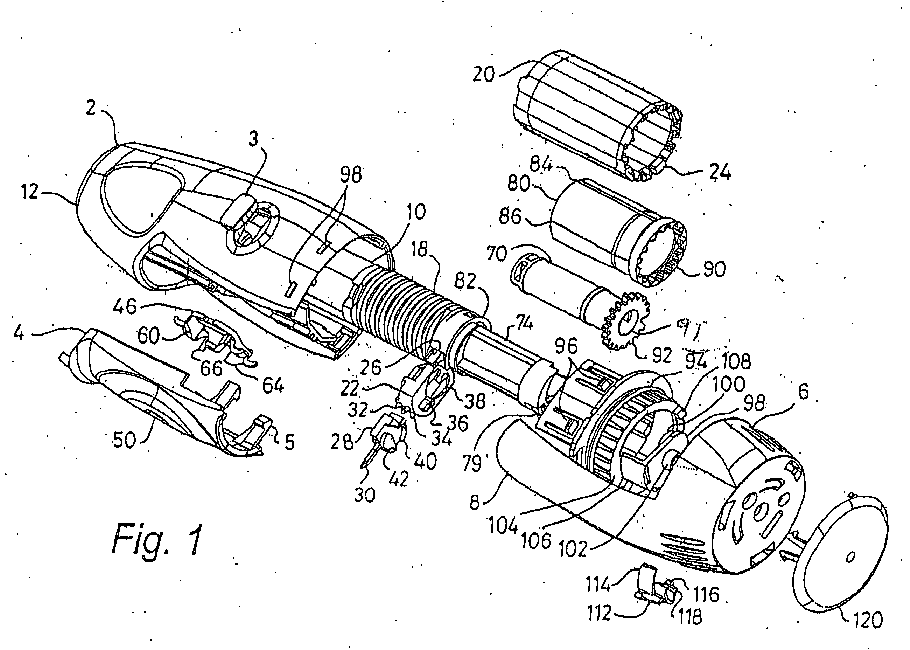

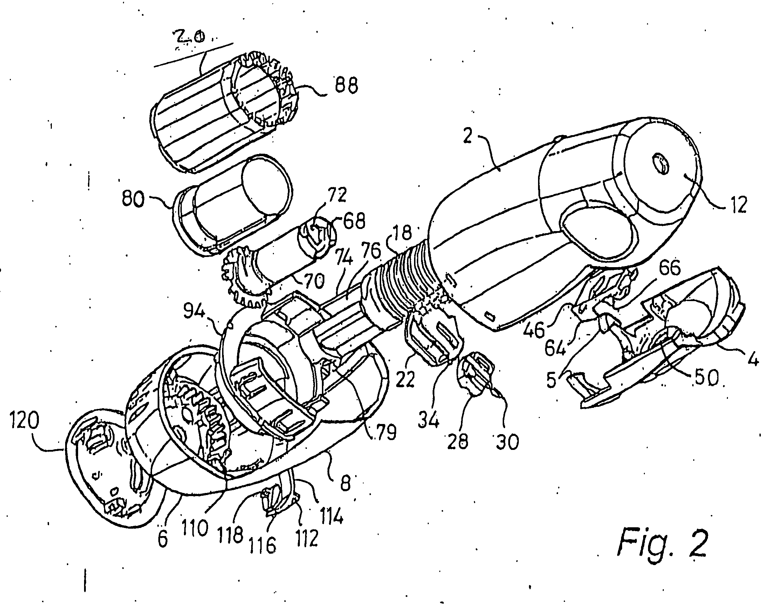

With reference to Figures 1 and 2, an inhaler comprises an elongate shell 2, at one side of which a mouthpiece 4 is attached. The inhaler includes a rotatable control member 6 which is situated at one end of the shell 2 and which incorporates a cover 8 for covering the mouthpiece 4 when the inhaler is not in use. The shell 2 is fitted with a window 3, through which a container (of medicament) in the inhaler can be viewed.

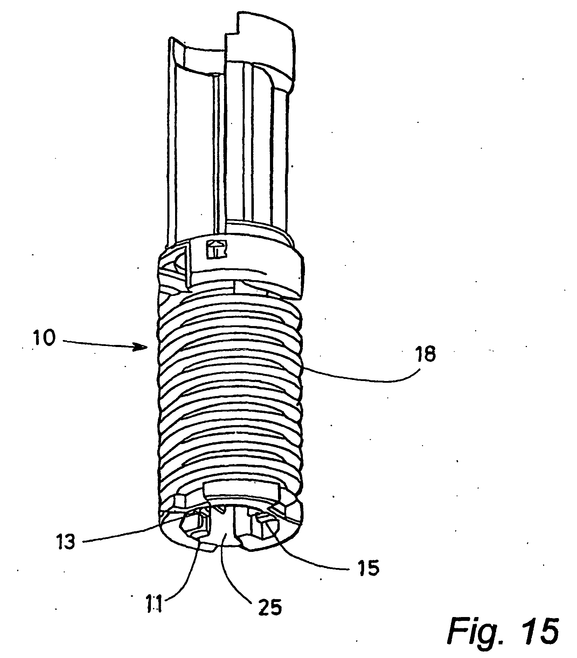

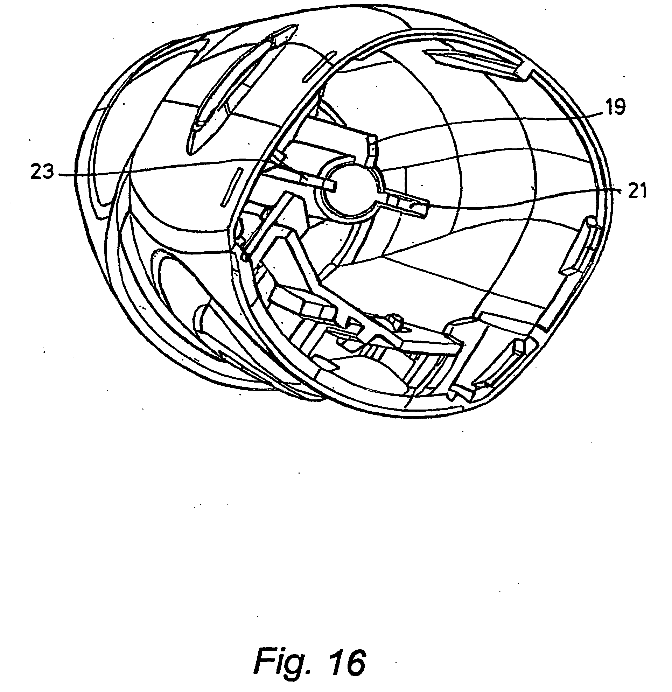

The shell accommodates an elongate hollow core 10 which is axially and radially fixed at one end to the end (denoted by reference numeral 12) of the shell 2. The core 10 is rotationally and axially fixed to the shell 2. As can be seen from Figure 15, the bottom of the core 10 is provided with three equi-angularly spaced slots 11, 13 and 15, each defined by a respective pair of opposed ribs which extend towards the core centre. As can be seen from Figure 16, the end of the shell 2 is provided with three ribs 19, 21 and 23. Each of the ribs 19, 21 and 23 extends a respective one of the slots 11, 13 and 15 in the core 10 (when the inhaler is assembled) and frictionally engage the ribs defining that slot. The frictional engagement between the ribs on the core 10 and the shell 2 retains the core 10 in and axially and rotationally fixes the core 10 to the shell 2. These formations leave clear an opening 25 in the bottom edge of the core to allow air to travel from an inlet (not shown) up through the core centre.

The core 10 has a bottom portion 18 which is externally screw-threaded and on which a cylindrical, dose carrying container 20 is mounted. The container carries a helical array of radial through bores, each of which contains a respective dose of powdered medicament, and is sealed by means of inner and outer laminated foil seals. A more detailed description of this type of container can be found in PCT publication No. WO 95/31238. The present container differs from a container as described in the earlier publication only in that the present container includes indentations (not shown) on its inner cylindrical surface for receiving an end of a locator device 22 as described below.

The container 20 has radial inward protuberances, for example 24, which engage the screw-thread of the bottom portion 18 such that rotation of the container 20 about the axis defined by the core 10 causes the container also to move axially along the core 10 to bring successive compartments into registry with the central portion of an opening 26 (that defines part of an airway in the inhaler) in the core 10. The opening 26 is an axial alignment with the locator 22 which is, in turn, slidably mounted in the core 10 so as to be movable in a direction perpendicular to the core axis. The locator 22 is hollow, has an end opening and slidably contains a pin holder 28 from which a U-section pin 30 extends. The locator 22 has four conical end projections, for example 32 and 34, which, in use, engage corresponding recesses on the inside surface of the container 20. One face of the locator 22 also carries a lug 36 positioned adjacent a generally C-shaped camming aperture 38 in a face of the locator 22.

The pin holder 28 is also provided with a key way 40 which is provided with a forward ramp 42 and is used in the extending and retracting of the pin 30 to rupture the seals on the compartments in the container 20.

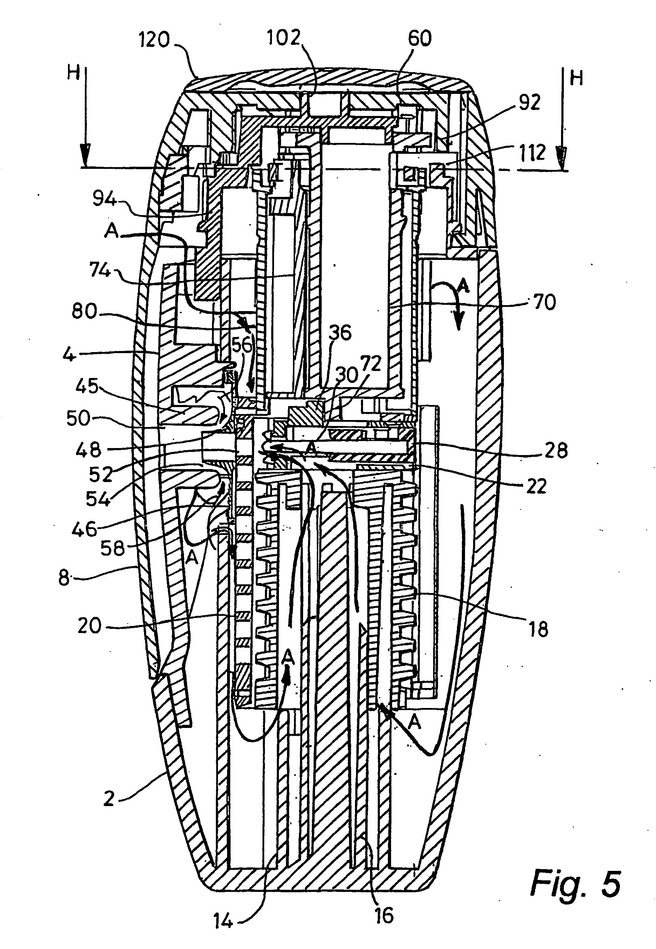

The pin 30 is axially aligned with a central passage, referenced 48 of an airway insert 46 which fits over a corresponding boss 45 on the mouthpiece 4. As can be seen from Figure 5, the airway insert 46 has a central passage 48 which extends into a corresponding passage 50 in the boss 45 of the mouthpiece 4, and which is in registry with a compartment (in this case the compartment 52) of the container 20. The portion of the insert 46 defining the passage 48 is spaced from the walls of the passage 50 to define an annular air inlet 54 for air flowing in directions indicated by the arrows 56 and 58. It will be seen from Figure 5 that the inlet 54 also constitutes a throat as it is narrower than both the upstream portions of the airway that feed it and than the passage 50.

The opening 26 in the core 10 provides the second air inlet which is situated behind a dose in the compartment 52. The insert 46 has four spacer lugs 60, 62, 64 and 66 which are equi-angularly arranged around the passage 50, and which extend generally radially relative to the core 10 and maintain the spacing between the insert 46 and the mouthpiece 4.

The locator 22 is extended by the action of a camming surface 68 (visible in Figure 2) which bears against the lug 36 and forms part of the end of the drive shaft 70. A peg 72 projects from the same end of the drive shaft 70 and is operable to engage the slot 38 to retract the locator 22. The peg also engages the key way 40 in the holder 28 to extend and retract the pin 30.

The core 10 has an upper portion 74 into which the drive shaft 70 is inserted. As can be seen from Figure 2, one side of the upper portion 74 includes a slot 76 which allows a pawl 78 on the drive shaft 70 to extend radially beyond the upper portion 74 when the pawl is in registry with the slot 76.

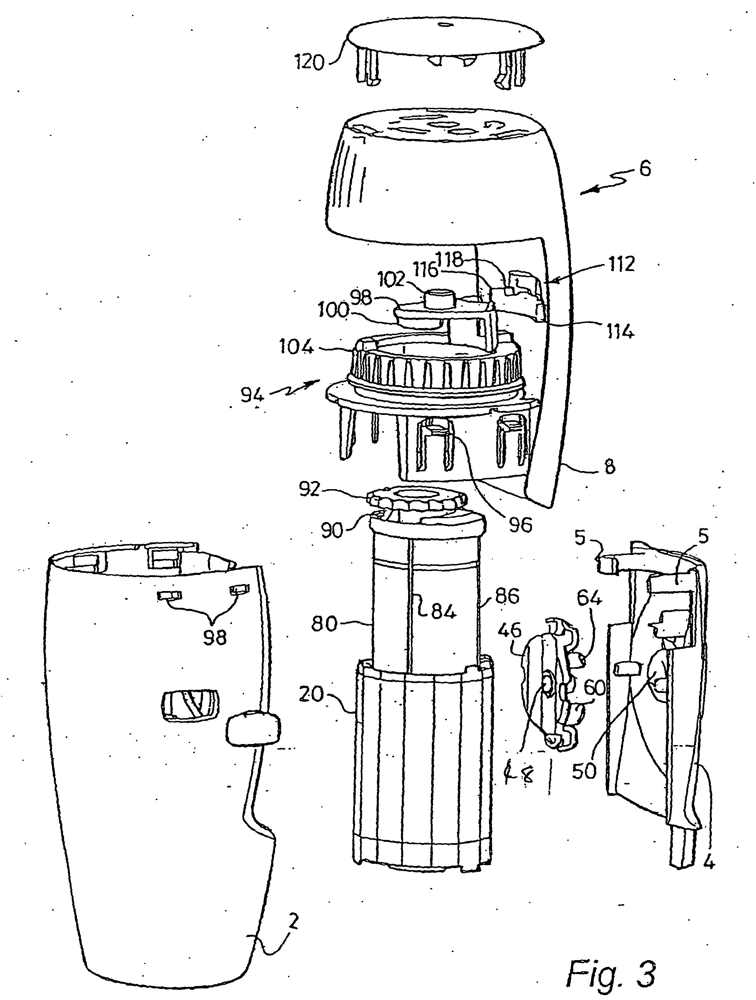

As can be seen from Figure 3, the upper portion 74 and shaft 70 both fit within a generally cylindrical index collar 80. The collar 80 is fitted onto the upper portion 74 before the shaft 70 is inserted, and is rotatably retained on the upper portion, and axially located by the annular shoulder 82 defined by the top of the lower portion 18 of the core 10.

A further pawl 79 is situated on the outside of the upper portion 74 at a position generally opposite the slot 76.

The index collar 80 has a series of longitudinal external slots, for example, 84 and 86 which engage corresponding inwardly directed lugs, for example 88, on the container 20. The relative dimensions of the container 20 and index collar 80 are such that the container 20 can slide along the outside of the index collar 80, but is rotationally fixed to the collar by the engagement of fixed lugs in the slots in the collar. Thus, rotation of the index collar 80 will cause a corresponding rotation of the container 20 which therefore also travels axially along the core 10 as a result of its engagement with the screw-threaded portion 18.

A ring gear 90 is provided at the end of the collar 80 opposite the end which rests on the shoulder 82. The teeth of the gears 90 are inwardly directed, and are, in use, engaged by the pawls 78 and 79. In the described embodiment, the shapes of the ends of the pawls and of the teeth are such that the pawls can be pulled over one tooth onto the next, but cannot readily be pushed in the opposite direction.

A gear wheel 92 is provided at the end of the drive shaft 70 in such a position as to protrude from the end of the sub-assembly of the core 10, container 20, indexing collar 80 and drive shaft 70.

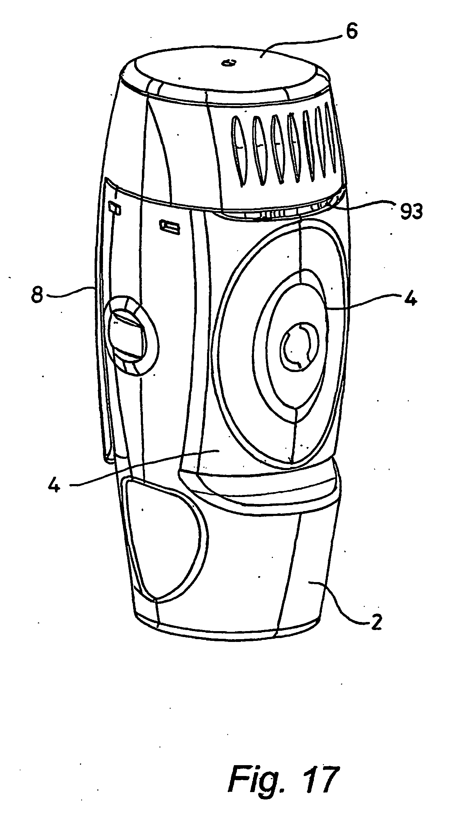

The mouthpiece 4 has latching components, for example 5, which engage in corresponding recesses/apertures in the shell 2 so that the mouthpiece 4 can be snap-fitted into position on the shell 2. With reference to Figure 17, an end of the mouthpiece 4 is spaced from the shell 2 and control member 6 to define an air inlet 93. When a user inhales through the mouthpiece 4, air travels through the inhaler from the inlet 93 to the mouthpiece 4 generally along the path indicated by the arrows A in Figure 5. As can be seen, air flows towards and through the opening 25, up the hollow interior of the core 10, and through the pin 30, locator 22 and dose cavity 52.

The sub-assembly is radially located by means of an inner cap 94 which has latching components 96 for engaging corresponding apertures 98 in the shell 2 to retain the cap 94 in position thereon. The cap 94 has an end stop 98 which carries a boss which is in axial alignment with the circular aperture 91 in the gear wheel 92. The end stop 98 also carries a second boss 102 the axis of which is spaced from that of the boss 100, and which extends in the opposite direction from the other boss. The inner cap 94 also incorporates an annular component 104, the outer surface of which carries a number of gear teeth. The component 104 also carries a pair of stops 106 and 108 which project axially from the end face of the component 104.

The member 6 is rotatably mounted on the boss 102 and incorporates a ring 110 (Figure 2) of inwardly directed teeth for meshing with the teeth on the gear wheel 92 of the shaft 70. A carrier device 112 is also mounted on the inside of the member 6, and carries a pair of oppositely directed pawls 114 and 116. The carrier 112 has an actuator 118 which projects radially inwards and (in use) engages either of the stops 106 or 108 (depending on the position of the member 6) to rock the carrier 112 so as to bring one or other of the pawls 114 and 116 into engagement with the teem on the annular component 104. An end piece 120 clips onto the member 6 to conceal tooling holes in the end of the latter.

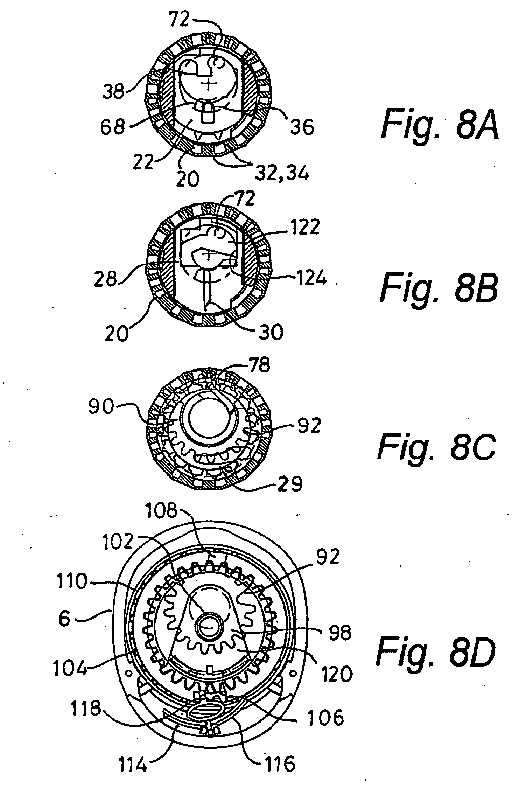

The member 6 is rotatable through approximately 180°, and one cycle of movement of the member comprises rotation in one direction about 180° and then rotation in the reverse direction through the same angle to return the member to its original position. This motion pierces the foil seal of a compartment 20 in registry with the pin 30 (and hence the airway defined by the insert 46 and mouthpiece 4), whilst uncovering the exit of the passage 50 to enable the user to inhale a dose through that exit, and then indexes the container 20 so that the next full compartment is in registry with the airway, and covers the mouthpiece. This cycle of operation will be described in more detail with reference to Figures 8-14.

Figures (8-14)A illustrate the effect of the rotation of the cover member 6 on the locator 22, Figures (8-14)B the effect of the same rotation on the pin holder 28 (and hence the pin 30), Figures (8-14)C the effect on the indexing mechanism, constituted by the indexing collar, the upper portion of the core 74 and the shaft 70, and Figures (8-14)D the effect on the non-return mechanism provided by the component 112 by the teeth on component 104 and the stops 106 and 108.

With the device in a start position shown in Figures 8A-D, the sealed, full compartment of the container 20 is in registry with the pin 30 and the airway defined by the passages 48 and 50. Thus, in order to make the contained dose available for inhalation, it is necessary to pierce the two sheets of foil which seal that compartment.

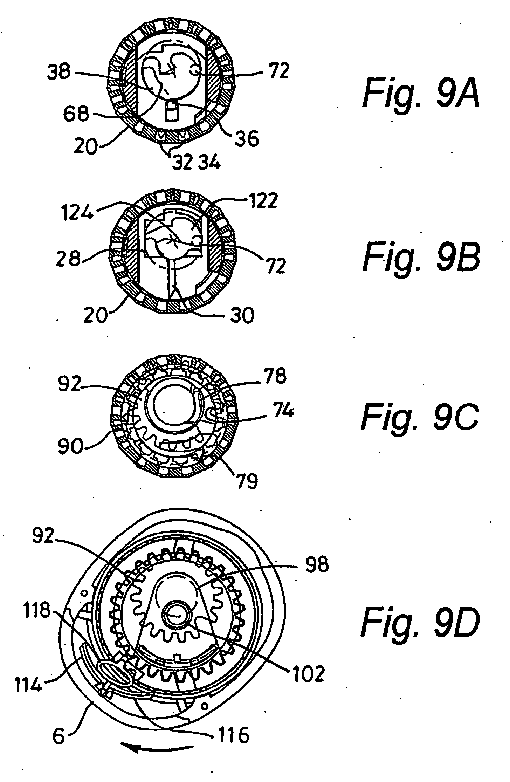

To that end, the control member 6 is rotated relative to the shell 2 in a clockwise direction as indicated in Figure 9D. This causes the ring of gear teeth 110 to rotate the gear wheel 92 and hence the shaft 70 in the same clockwise direction. The rotation of the shaft 70 brings the camming surface 68 on the base of that shaft into engagement with the lug 36 on the locator 22, causing the locator to extend into the position shown in Figure 9A, in which the cones (for example 32 and 34) on the end of the locator 22 extend into corresponding recesses in the container 20, firmly to locate the compartment relative to the pin 30 During this phase of movement, the peg 72 passes along a circumferential portion (122 in Figures 8B and 9B), which corresponds to the arc of movement of the peg 72. As a result, the pin 30 remains retracted within the core 10 during this first phase of operation of the device. -

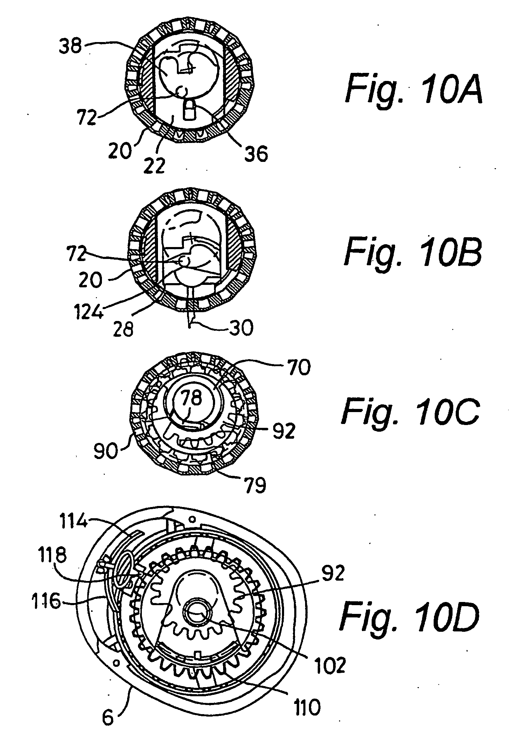

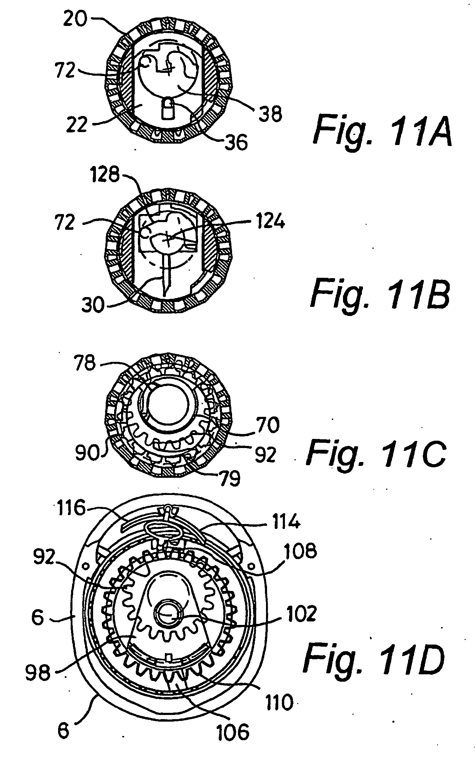

Figure 9B shows the peg when it has reached a non-circumferential portion 124 of the key way 40. Consequently, further rotation of the cover 8 in the same direction will then extend the pin 30 as shown in Figure 10B. This movement of the pin 30 causes it to travel through the compartment, and thus to pierce both foil seals on either side of the compartment. The sectional shape of the pin 30 is such that this movement does not eject any significant amount of the material to be inhaled from the compartment. The initial rotation of the control member 6 causes the pawl 78 on the shaft 70 to be withdrawn into the upper portion 74 of the core 10 so that it cannot engage the teeth 90 on the indexing collar 80. It can also be seen from Figures 8D, 9D and 10D that the pawl 116 engages the ring of teeth 110 on the inner cap 94. The pawl 116 thus allows the rotation of the control member 6 in an anti-clockwise direction, but prevents rotation in the opposite sense. The continuing anti-clockwise rotation of the control member 6 into the position shown in Figure 11D causes the peg 72 to engage a further surface 126 of the key way 40, and thus to withdraw the pin 30 from the compartment. Again, the shape of the pin 30 is such that its withdrawal does not remove any substantial amount of particulate material from the compartment. During this movement of the control member 6, the camming surface 68 continues to hold the locator 22 in engagement with the container 20, and the upper portion 76 of the core 10 continues to keep the pawl 78 out of engagement with the teeth 90 of the indexing collar 80. As the cover 8 has been rotated to the opposite side of the shell 2 from the mouthpiece 4, the exit 50 is at this stage accessible to a user who can inhale the dose of material from the compartment. Inhalation by the user through the mouthpiece 4 creates a stream of air flowing into the passage 50 through the annular inlet 54. The airway insert and passage 50 define between them a throat which accelerates this flow of air, thus creating an area of low pressure in front of the passage 48, and hence the dose in the compartment 52, and this helps to establish a stream of air flowing through the compartment 52 and into the passage 50, in which stream of air the dose is entrained. As the dose leaves the ejection zone, (defined in this case by the compartment 52 and passage 48) the air flowing in through the inlet 54 forms a jacket which prevents the entrained dose from significantly impinging on the walls of the passage 50.

As can be seen from Figure 11D, the actuator 118 of the carrier.112 has been rocked by the stop 108 so as to bring the pawl 114 into engagement with the teeth and to disengage the pawl 116. Since the pawl 116 is now disengaged, the control member can be rotated in the opposite sense (i.e. clockwise), but the pawl 114 will prevent anti-clockwise rotation until the control member 6 has been returned to its start position.

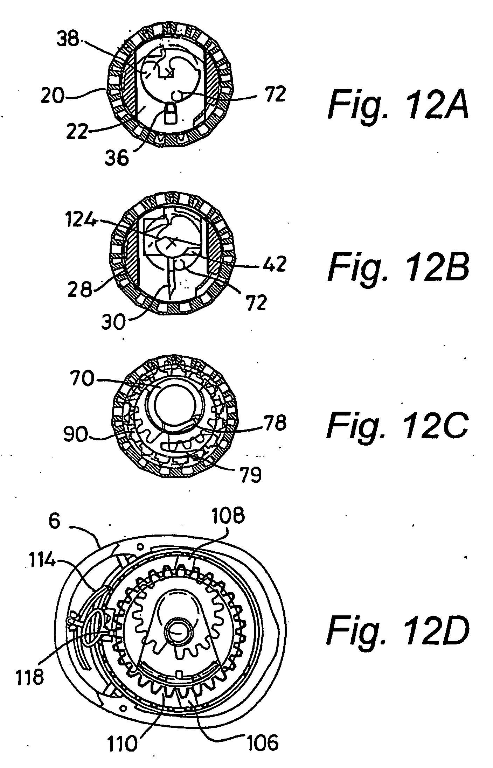

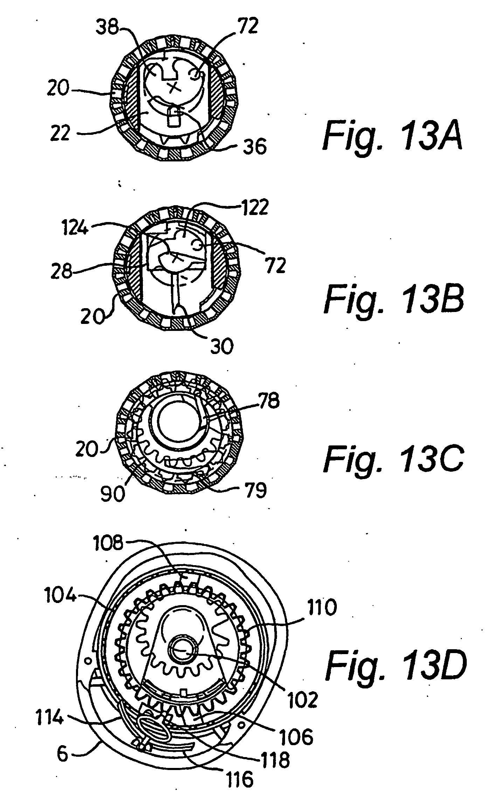

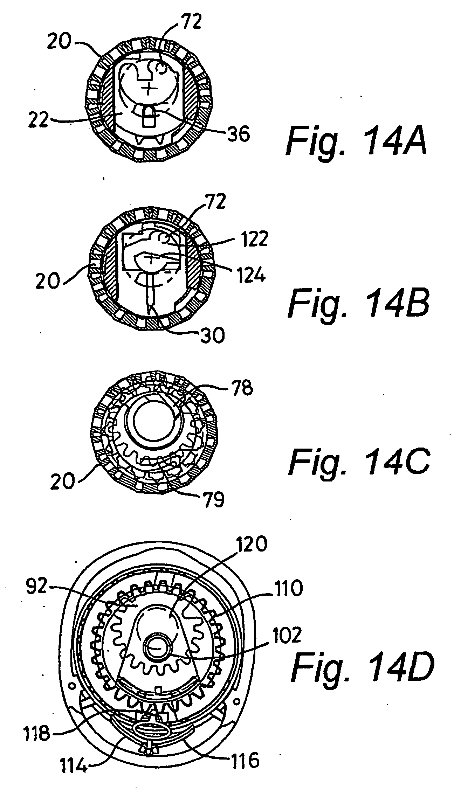

With reference to Figures 12A-D, as the control member 6 returns to its start position, the shaft 70 rotates within the core 10 to move the pawl 78 towards the slot 76. In addition, the peg 72 passes in front of the pin holder 28 and towards the inclined ramp 42. Continued clockwise rotation of the control member 6 moves the camming surface 68 out of engagement with the lug 36 and the peg 72 into engagement with the camming aperture 38, and thus causes the locator 22 to be withdrawn back into the core 10. The movement also causes the pawl 78 to extend out of the slot 76 and into engagement with one of the teeth 90 in the indexing collar 80 (Figure 13C). Continued rotation of the control member 6 then causes the pawl 78 to push the indexing collar 80 in an anti-clockwise direction as viewed from Figure 13C, thus allowing the pawl 79 to ride over a tooth of the gear 90. This rotation of the indexing collar 80 correspondingly rotates the container 20, and moves the container in a small axial direction towards the inner cap 94 by virtue of the screw-threaded engagement with the portion 18. Thus, the container 20 is indexed into the next position in which the next compartment is in registry with the pin 30 and the airway 48. Figure 14D shows the cover member when it is close to its original position, at which stage the actuator 118 engages the stop 106 to move the carrier 112 back to its original position (in which it is the pawl 116 that engages the teeth 94). It will be appreciated that the pawl 79 stops the collar 80 (and hence the container 20) rotating as the control member is moved in a clockwise direction, whilst allowing movement of the member in the other direction to index the container 20.

In addition, since the gear wheel 92 is of a smaller diameter than the ring of teeth 110, a rotation of 180 of the control member 6 causes the shaft 70 to rotate through a larger angle, thus enabling the peg 76 both to extend and withdraw the pin 30 in response to the rotation of the cover member from the start position to the position shown in Figure 11D. In this particular example, that movement of the cover member constitutes an advanced stroke, whilst the return, anti-clockwise movement position shown in Figure 8D is a return stroke of the cover member 6.

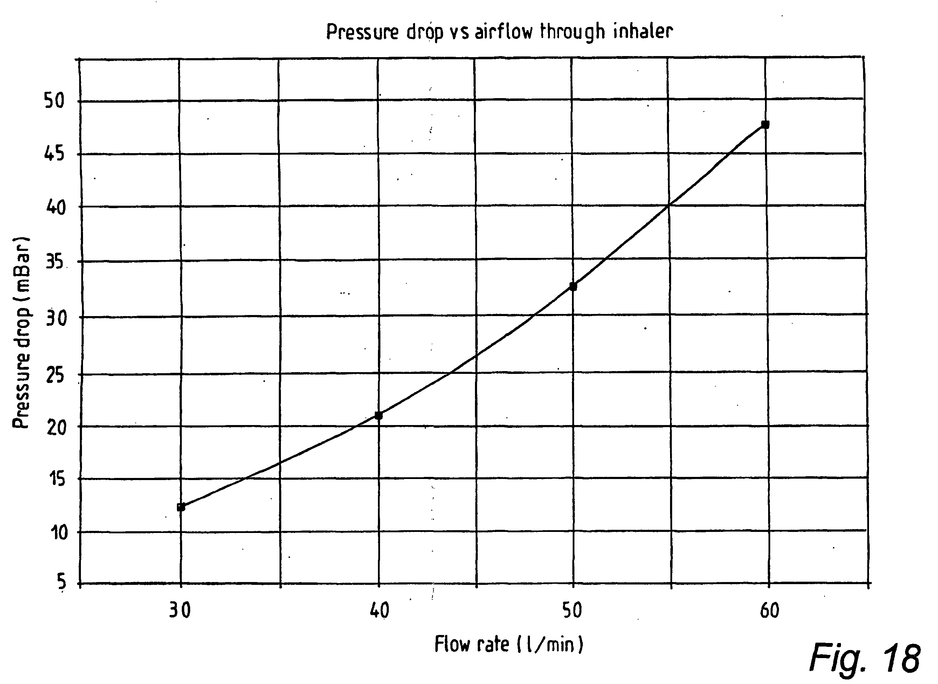

Figure 18 is a graph illustrating the relationship between the pressure drop along the passage 48 and the total rate of flow of air through the inhaler. The graph shows that even low flow rates provide a significant pressure drop. The inhaler design thus helps to ensure that a full dose of powder is inhaled even if the user is unable to inhale properly.

## Claims

1. A device for dispensing a plurality of doses of particulate material, the device comprising receiving means (10, 80) for receiving and retaining a container (20) having a plurality of compartments, each for holding a respective dose or part thereof, the device further comprising an airway (48, 50) extending from the receiving means to an outlet (4), indexing means (18, 80, 78, 79, 90) for moving a container (20) in the receiving means (10, 80) relative to the airway (48, 50) so as to bring successive compartments into registry with the latter, a control member (6) movable through a series of alternating advance and return strokes to operate the indexing means (18, 80; 78, 79, 90), **characterised in that** the device includes a non-return mechanism (94, 104, 106, 108, 112, 114, 116, 118) for preventing movement of the member (6) in the direction of each return stroke before the completion of the respective preceding advance stroke.

2. A device according to claim 1, in which the control member (6) is so linked to the indexing means (18, 80, 78, 79, 90) that, in use, said movement of the container (20) received by the receiving means is caused by the return strokes of the control member (6).

3. A device according to claim 1 or claim 2, in which the device is adapted for use with a container (20) in which each dose is sealed in its compartment, the device including opening means (30) for breaking the seal on each compartment, wherein the opening means is also linked to the control member (6) so that operation of the latter operates both the indexing means and the opening means (30).

4. Apparatus according to claim 3, in which the opening means comprises a piercing member (30) which is so linked to the control member (6) that each advance stroke of the latter extends a piercing member (30) from a retracted position, in which it is positioned clear of a compartment, into an extended position in which it has pierced that compartment's seal.

5. A device according to any of the preceding claims, in which the device includes a holding member (22) so linked to the control member (6) as to be extended by each advance stroke of the latter to engage a container (20) held in the device and thereby hold a compartment in registry with the airway (48, 50).

6. A device according to any of the preceding claims, in which the non-return mechanism (94, 104, 106, 108, 112, 114, 116, 118) is also operable to prevent movement of the control member (6) in the direction of each advance stroke until the end of the respective preceding return stroke has been reached.

7. A device according to any of the preceding claims, in which the advance and return strokes of the control member (6) are constituted by rotational movements of the latter relative to a body of the device, the non-return mechanism comprising a pawl means (112, 114, 116) and a set of teeth (110), each mounted on a respective one of the control member (6) and the body (2).

8. A device according to claim 7 when appended to claim 6, in which the pawl means (112, 114, 116) comprises a pair of opposed pawls (114, 116) mounted on a movable carrier (112), one pawl engaging the teeth on each advance stroke, the other on each return stroke, the mechanism including abutment means (106, 108) for moving one pawl into engagement with the teeth and disengaging the other pawl at the end of each stroke.

9. A device according to any of the preceding claims, in which the device comprises an inhaler for dispensing doses of powdered medicament.

10. A housing of a device according to any of the preceding claims, the housing having said receiving means (10, 80) for receiving said container (20) of particulate material, means (19, 21, 23) for receiving said indexing means (18, 80, 78, 79,90), an outlet for said airway of the container, the housing also carrying said control member (6) for operating the indexing means (18, 80, 78, 79, 90), and containing means (110) for linking the control member (6) to an indexing means (18, 80, 78, 79, 90), in use, contained within the housing, **characterised in that** the housing includes the non return mechanism (94, 104, 106, 108, 112, 114, 116, 118) for preventing movement of the control member (6) in the direction of each return stroke before the completion of the respective preceding advance stroke.

## Patentansprüche

1. Vorrichtung zum Verabreichen einer Vielzahl von Dosen in Partikelform, wobei die Vorrichtung ein Aufnahmemittel (10, 80) zum Aufnehmen und Zurückhalten eines Behälters (20) umfasst, der eine Vielzahl von Fächern hat, jedes zum Halten einer jeweiligen Dosis oder eines Teils davon, wobei die Vorrichtung ferner einen Luftweg (48, 50) umfasst, der sich von dem Aufnahmemittel zu einem Auslass (4) erstreckt, Indexierungsmittel (18, 80, 78, 79, 90) zum Bewegen eines Behälters (20) in dem Aufnahmemittel (10, 80) relativ zu dem Luftweg (48, 50), um aufeinanderfolgende Fächer in Zug mit Letzterem zu bringen, ein Steuerelement (6), das durch eine Reihe abwechselnder Vorwärts- und Rückwärtshübe bewegbar ist, um das Indexierungsmittel (18, 80, 78, 79, 90) zu betätigen, **dadurch gekennzeichnet, dass** die Vorrichtung einen Rücklaufschutzmechanismus (94, 104, 106, 108, 112, 114, 116, 118) enthält, um das Bewegen des Steuerelements (6) in die Richtung jedes Rücklaufhubs zu verhindern, bevor der jeweilige vorhergehende Vorwärtshub abgeschlossen ist.

2. Vorrichtung nach Anspruch 1, bei der das Steuerelement (6) so mit dem Indexierungsmittel (18, 80, 78, 79, 90) verbunden ist, dass die Bewegung des Behälters (20), der von dem Aufnahmemittel aufgenommen wird, beim Gebrauch durch die Rückwärtshübe des Steuerelements (6) verursacht wird.

3. Vorrichtung nach Anspruch 1 oder 2, bei der die Vorrichtung für den Gebrauch mit einem Behälter (20) geeignet ist, bei dem jede Dosis in ihrem Fach versiegelt ist, wobei die Vorrichtung Öffnungsmittel (30) zum Durchstoßen der Versiegelung auf jedem Fach enthält, wobei das Öffnungsmittel auch mit dem Steuerelement (6) verbunden ist, so dass das Betätigen dieses Letzteren sowohl das Indexierungsmittel als auch das Öffnungsmittel (30) betätigt.

4. Vorrichtung nach Anspruch 3, bei dem das Öffnungsmittel ein Durchstoßelement (30) umfasst, das so mit dem Steuerelement (6) verbunden ist, dass jeder Vorwärtshub dieses Letzteren ein Durchstoßelement (30) aus einer zurückgezogenen Stellung, in welcher es von einem Fach beabstandet positioniert ist, in eine ausgestreckte Stellung streckt, in der es die Versiegelung des Fachs durchstoßen hat.

5. Vorrichtung nach einem der vorhergehenden Ansprüche, bei der die Vorrichtung ein Halteelement (22) umfasst, das so mit dem Steuerelement (6) verbunden ist, dass es durch jeden Vorwärtshub dieses Letzteren ausgestreckt wird, um in einen Behälter (20) einzugreifen, der in der Vorrichtung gehalten wird und **dadurch** ein Fach in Zug mit dem Luftweg (48, 50) zu halten.

6. Vorrichtung nach einem der vorhergehenden Ansprüche, bei der der Rücklaufschutzmechanismus (94, 104, 106, 108, 112, 114, 116, 118) auch betätigt werden kann, um das Bewegen des Steuerelements (6) in die Richtung jedes Vorwärtshubs zu verhindern, bis das Ende des jeweiligen vorausgehenden Rückwärtshubs erreicht ist.

7. Vorrichtung nach einem der vorhergehenden Ansprüche, bei dem der Vorwärts- und der Rückwärtshub des Steuerelements (6) aus Drehbewegungen des Letzteren zu einem Körper der Vorrichtung bestehen, wobei der Rücklaufschutzmechanismus ein Klinkenmittel (112, 114, 116) und einen Satz von Zähnen (110) umfasst, die jeweils entweder auf das Steuerelement (6) oder auf den Körper (2) montiert sind.

8. Vorrichtung nach Anspruch 7, wenn angehängt an Anspruch 6, bei der das Klinkenmittel (112, 114, 116) ein Paar entgegengesetzter Klinken (114, 116) umfasst, die auf einen beweglichen Träger (112) montiert sind, wobei eine Klinke bei jedem Vorwärtshub in die Zähne eingreift, die andere bei jedem Rückwärtshub, wobei der Mechanismus ein Anschlagmittel (106, 108) umfasst, um eine Klinke in das Eingreifen mit den Zähnen zu bewegen und die andere Klinke am Ende jedes Hubs zu lösen.

9. Vorrichtung nach einem der vorhergehenden Ansprüche, bei der die Vorrichtung einen Inhalator zum Verabreichen von Dosen von Arzneimitteln in Partikelform umfasst.

10. Gehäuse einer Vorrichtung nach einem der vorhergehenden Ansprüche, wobei das Gehäuse das Aufnahmemittel (10, 80) zum Aufnehmen des Behälters (20) von Stoff in Partikelform hat, Mittel (19, 21, 23) zum Aufnehmen des Indexierungsmittels (18, 80, 78, 79, 90), einen Auslass für den Luftweg des Behälters, wobei das. Gehäuse auch das Steuerelement (6) zum Betätigen des Indexierungsmittels (18, 80, 78, 79, 90) und Aufnahmemittel (110) zum Verbinden des Steuerelements (6) mit einem Indexierungsmittel (18, 80, 78, 79, 90) beim Gebrauch, das in dem Gehäuse enthalten ist, **dadurch gekennzeichnet, dass** das Gehäuse den Rücklaufschutzmechanismus (94, 104, 106, 108, 112, 114, 116, 118) umfasst, um das Bewegen des Steuerelements (6) in die Richtung jedes Rücklaufhubs zu verhindern, bevor der jeweilige vorhergehende Vorwärtshub abgeschlossen ist.

## Revendications

1. Dispositif pour administrer une pluralité de doses d'une substance particulaire, le dispositif comprenant des moyens de réception (10, 80) pour recevoir et loger un réservoir (20) présentant une pluralité de compartiments contenant chacun une dose respective ou une partie de celle-ci, le dispositif comprenant en outre une voie d'air (48, 50) s'étendant à partir des moyens de réception jusqu'à une sortie (4), des moyens de repérage (18, 80, 78, 79, 90) pour déplacer un réservoir (20) dans les moyens de réception (10, 80) par rapport à la voie d'air (48, 50) de manière à amener les compartiments successifs en alignement avec cette dernière, un élément de commande (6) mobile à travers une série de courses vers l'avant et vers l'arrière pour actionner le moyen de repérage (18, 80, 78, 79, 90), **caractérisé en ce que** le dispositif comprend un mécanisme de non retour (94, 104, 106, 108, 112, 114, 116, 118) destiné à empêcher tout déplacement de l'élément (6) dans la direction de chaque course vers l'arrière avant la fin de la course vers l'avant précédente respective.

2. Dispositif selon la revendication 1, dans lequel l'élément de commande (6) est relié aux moyens de repérage (18, 80, 78, 79, 90) de telle sorte que, lors de l'utilisation, ledit déplacement du réservoir (20) reçu par les moyens de réception est provoqué par les courses vers l'arrière de l'élément de commande (6).

3. Dispositif selon la revendication 1 ou la revendication 2, dans lequel le dispositif est adapté pour être utilisé avec un réservoir (20) dans lequel chaque dose est isolée dans son compartiment, le dispositif comportant un moyen d'ouverture (30) pour casser le joint de chaque compartiment, dans lequel le moyen d'ouverture est également relié à l'élément de commande (6) de telle sorte que l'actionnement de ce dernier actionne à la fois les moyens de repérage et le moyen d'ouverture (30).

4. Dispositif selon la revendication 3, dans lequel le moyen d'ouverture comprend un élément de perforation (30) relié à l'élément de commande (6) de telle sorte que chaque course vers l'avant de ce dernier étende un élément de perforation (30) à partir d'une position rétractée dans laquelle il est positionné hors d'un compartiment, dans une position étendue dans laquelle elle perfore le joint de ce compartiment.

5. Dispositif selon l'une quelconque des revendications précédentes, dans lequel le dispositif comprend un élément de support (22) relié à l'élément de commande (6) de manière à être étendu lors de chaque course vers l'avant de ce dernier pour engager un réservoir (20) logé dans le dispositif et supporter ainsi un comportement en alignement avec la voie d'air (48, 50).

6. Dispositif selon l'une quelconque des revendications précédentes, dans lequel le mécanisme de non retour (94, 104, 106, 108, 112, 114, 116, 118) peut également être actionné pour empêcher tout déplacement de l'élément de commande (6) dans la direction de chaque course vers l'avant jusqu'à ce que la fin de la course vers l'arrière précédente respective ait été atteinte.

7. Dispositif selon l'une quelconque des revendications précédentes, dans lequel les courses vers l'avant et vers l'arrière de l'élément de commande (6) sont constituées par des mouvements rotatifs de ce dernier par rapport à un corps du dispositif, le mécanisme de non retour comprenant des moyens de cliquet (112, 114, 116) et un ensemble de dents (110), montés chacun sur un élément respectif parmi l'élément de commande (6) et le corps (2).

8. Dispositif selon la revendication 7, lorsqu'elle est associée à la revendication 6, dans lequel les moyens de cliquet (112, 114, 116) comprennent une paire de cliquets opposés (114, 116) montés sur un support mobile (112), un premier cliquet engageant les dents lors de chaque course vers l'avant, l'autre cliquet faisant de même lors de chaque course vers l'arrière, le mécanisme comprenant des moyens de butée (106, 108) pour déplacer un premier cliquet en engagement avec les dents et désengager l'autre cliquet à la fin de chaque course.

9. Dispositif selon l'une quelconque des revendications précédentes, dans lequel le dispositif comprend un inhalateur pour administrer des doses d'un médicament en poudre.

10. Boîtier d'un dispositif selon l'une quelconque des revendications précédentes, le boîtier comprenant lesdits moyens de réception (10, 80) destinés à recevoir ledit réservoir (20) de substance particulaire, des moyens (19, 21, 23) pour recevoir lesdits moyens de repérage (18, 80, 78, 79, 90), une sortie pour ladite voie d'air du réservoir, le boîtier portant aussi ledit élément de commande (6 ) pour actionner les moyens de repérage (18, 80, 78, 79, 90), et contenant des moyens (110) pour relier l'élément de commande (6) aux moyens de repérage (18, 80, 78, 79, 90), lors de l'utilisation, logés dans le boîtier, **caractérisé en ce que** le boîtier comprend le mécanisme de non retour (94, 104, 106, 108, 112, 114, 116, 118) destiné à empêcher tout déplacement de l'élément de commande (6) dans la direction de chaque course vers l'arrière avant la fin de la course vers l'avant précédente respective.
